# EUROPEAN PATENT SPECIFICATION

(11) **EP 2 131 681 B1**
(45) Date of publication and mention of the grant of the patent: **12.12.2012**
(21) Application number: 08738428.5
(22) Date of filing: 28.02.2008
(51) Int. Cl.: A23L 1/30, A61K 36/28, B01D 61/02, B01D 61/14, B01D 61/58, A61K 8/97, A61Q 19/00, B01D 63/10

(54) **PROCESS FOR PRODUCING REFINED NUTRACEUTIC EXTRACTS FROM ARTICHOKE WASTE AND FROM OTHER PLANTS OF THE CYNARA GENUS**
VEFAHREN ZUR HERSTELLUNG RAFFINIERTER NUTRAZEUTISCHER EXTRAKTE AUS ARTISCHOCKENRESTEN UND ANDEREN DISTELGEWÄCHSEN
PROCÉDÉ DE PRODUCTION D'EXTRAITS NUTRACEUTIQUES RAFFINÉS À PARTIR DES DÉCHETS D'ARTICHAUT ET À PARTIR D'AUTRES PLANTES DU GENRE CYNARA

(30) Priority: 28.02.2007 IT RM20070109
(43) Date of publication of application: 16.12.2009
(73) Proprietor: Isr Ecoindustria S.r.l., 04100 Latina (IT); Pizzichini, Massimo, 00123 Roma (IT); Romani, Annalisa, 51031 S.Michele Agliana (IT)
(72) Inventor: PIZZICHINI, Massimo, I-00123 Roma (IT); ROMANI, Annalisa, I-51031 S.Michele Agliana (IT); PIZZICHINI, Daniele, I-00123 Roma (IT); RUSSO, Claudio, I-00186 Roma (IT); PINELLI, Patrizia, I-50019 Sesto Fiorentino (IT)
(74) Representative: Banchetti, Marina
(86) International application number: PCT/IT2008/000135
(87) International publication number: WO 2008/105023

(56) References cited:
- EP-A- 1 378 491
- US-A- 4 083 779
- US-A- 5 780 060
- US-A1- 2004 234 674
- US-B1- 6 818 234
- RAFAEL LLORACH, JUAN CARLOS ESPÍN, FRANCISCO A. TOMÁS-BARBERÁN, FEDERICO FERRERES: "Artichoke (Cynara scolymus L.) Byproducts as Potential Source of Health-Promoting Antioxidant Phenolics" JOURNAL OF AGRICULTURAL AND FOOD CHEMISTRY, vol. 50, 2002, pages 3458-3464, XP002484412
- RAFAEL LLORACH, FRANCISCO A. TOMÁS-BARBERÁN, FEDERICO FERRERES: "Functionalisation of commercial chicken soup with enriched polyphenol extract from vegetable by-products" EUROPEAN FOOD RESEARCH AND TECHNOLOGY, vol. 220, 2005, pages 31-36, XP002484413

## Description

The present invention concerns a process for producing refined nutraceutic extracts from artichoke waste and from other plants of the *Cynara* genus. More specifically, the invention concerns a process for fractioning and refining natural products obtainable from waste vegetal material, and particularly the waste of artichoke production, or from other vegetal material coming from the *Cynara* genus, such as the cardoon. The process is based on the use of membrane-based separation technologies and enables producing purified extracts of high biological valence to be used in the pharmaceutical industry, in the nutraceutical field, in the cosmetics industry or for innovative products in the food industry.

As is known, the *Cynara* genus, which belongs to the *Compositae* family, includes the species *Cynara cardunculus* which in turn includes the artichoke (*C*. *cardunculus* L. subsp. *scolymus* L., normally referred to as *Cynara scolymus*), the cultivated cardoon (*C*. *cardunculus* L. var. *altilis* DC), the wild cardoon (*C*. *cardunculus* L. var. *sylvestris* Lam.) and some other lesser known species. The inflorescence of these plants, the heads, are made up of many single flowers, of a purplish colour when blooming, which are gathered in the central part by a receptacle or thalamus, around the outermost part of which there are the bracts or involucral scales.

When they are immature and tender, the heads of the *Cynara scolymus* are harvested, before the bracts harden and before the central flowers grow too much. They provide the edible part of the artichoke, commonly known as the "heart", consisting of the receptacle and the florets it contains, as well as of the fleshy bases of the internal bracts, and possibly of a part of the peduncle.

The artichoke is a characteristic Italian product, although in recent years its cultivation has also extended to other geographic areas (such as California, Argentina and New Zealand), and it has been estimated that the Mediterranean area has 90% of the world's production, with Italy being the leading producer with an estimated production well over 500,000 tons/year. Although Italy has considerable land given over to artichoke production, there are also notable productions in Spain and France.

Italian production is mainly destined for fresh consumption, even if the quantity destined for the food processing industry is growing, especially as regards the production of oil-preserved foodstuffs.

Unlike many other cultivations, in artichoke growing the relation between the edible part and the whole plant is extremely low (about 15%), and thus the quantity of by-product is considerable. The artichoke heart accounts for 40-55% of the weight of the whole head and contains about 15% of dry matter, and is particularly rich in fibre. The remaining part, consisting of leaves and stems, constitutes the waste material that must often be discarded with additional waste treatment costs in compliance with environmental laws. This waste matter could be widely and advantageously used both as a semi-processed component in human foods and as animal feed. The use of waste matter and residues deriving from the industrial agronomic sector of artichoke production and its derivatives could enable a reduction in the environmental impact of the production itself, and the reduction of labour costs and biomass disposal costs.

Although the current state of the art is essentially geared to their conversion into energy, rarely are these biomasses considered to represent new low-cost raw materials for the production of bio-products of high added value for the food, phytotherapy and cosmetics industry.

The total raw fibre of the artichoke, consisting of 65% cellulose, 21 % semi-cellulose and 14% lignin, accounts for an overall 1.4% of the fresh substance. From a nutritional and dietary point of view, the artichoke is a noble food for the abundance of azotised substances and for its carbohydrate content. In essence, it is a vegetable of average energy value (38 cal/100 g), not only rich in mineral salts (K, Na, Ca, P, Fe), vitamins (A, B₁, C) and sugars (including inulin, a fructose polymer of pre-biotic activity, which promotes the proper equilibrium of bowel bacterial flora), but also - and especially - polyphenol compounds responsible for considerable biological activity for which the artichoke has been renowned as an officinal plant since ancient times.

In fact, artichoke extracts and dyes, which are mostly obtained from the plant's leaves, have long been used as hepatoprotectors and regulators of liver functioning, in hepatobiliary pathologies, as choleretics (to increase biliary flow) and hypocholesterolemics (to lower cholesterol levels in the blood). In general, both the spontaneous and cultivated forms of the *Cynara* genus are used in order to extract biopharmaceutical substances.

The different compounds of vegetable origin contained in them can express different biological properties (antioxidant, anti-radical, antimicrobial) and, in many cases, the single molecule is less active with respect to the mixture of compounds, thus suggesting a synergic action among them. In other words, it is not rare to find that raw extracts of vegetable origin have a higher bioactivity with respect to the one found when individual compounds in these plants are used alone.

The compounds responsible for the biological activities of the artichoke include: **a**) the hydroxycinnamates of the chlorogenic acid family, among which cholorogenic or mono-caffeoylquinic acid (5-O-caffeoylquinic), dicaffeoylquinic or cynarin acid (1,5-dicaffeoylquinic acid); **b**) flavonoids and flavonoic eterosides (luteolin and its conjugates, such as cynaroside (luteolin-7-O-glucoside), scolimoside, luteolin-glucuronide; **c**) lactone sesquiterpenes, among which the compounds responsible for the characteristic bitter taste, cynaropicrin and dehydrocynaropicrin.

The *Cynara* thus has important applications not only in phytotherapy but also in the cosmetics industry, where the extracts are used for their antioxidant and anti-free radical properties.

In many pharmacological studies, the *Cynara* extracts have been found to possess various properties: (i) they protect proteins, lipids and DNA from oxidation caused by free radicals; (ii) they show a choleretic, diuretic and hepatoprotective activity; (iii) they inhibit the biosynthesis of cholesterol contributing to the prevention of arteriosclerosis and other circulatory disorders; (iv) they inhibit HIV integrase, the key enzyme in HIV replication and in its integration in the host genoma; and (v) they possess antibacterial activity.

Recent studies (Romani A., Pinelli P., Cantini C., Cimato A. & Heimler D. "Characterization of Violetto di Toscana, a typical Italian variety of artichoke (Cynara scolymus L.)", Food Chemistry, 95, 221-225, 2006) on cultivars of Violetto di Toscana and Terom, the latter widely available on the market, have characterised the polyphenol content of various *Cynara* tissues (leaves, bracts, heads, stems) and it has been found that the stem has a polyphenol composition very similar to that of the head, that is, the edible part of the artichoke. These tissues are essentially rich in caffeoylquinic derivatives, while the leaves and bracts are also rich in flavonoid compounds.

The antioxidant properties in human LDL (low density lipoproteins) of extracts obtained from various artichoke tissues have also been evaluated (Coinu R., Carta S., Urgeghe P. P., Mulinacci N., Pinelli P., Franconi F., Romani A. "Dose-Effect study on the antioxidant properties of leaves and outer bracts of extracts obtained from Violetto di Toscana artichoke", Food Chem., 2007, 101, 524-531) for which a high activity has always been recorded. The studies have also been extended to cultivated cardoon and its progenitor, the wild cardoon, both belonging to the *Cynara* genus. These species contain polyphenol antioxidants similar to those found in the artichoke (caffeoylquinic acids and flavonoids) (Pinelli P., leri F., Buzzini P., Turchetti B., Lanteri S., Romani A., "Composti polifenolici ad attività antimicotica in tessuti di diverse cultivar di carciofo", VII CISETA (Congresso Italiano di Scienza E Tecnologia degli Alimenti), Cernobbio (CO), 19-20 Settembre 2005). The cardoon also contains hydroxycinnamic compounds which are either absent or found only in traces in the artichoke (Pinelli P., Agostini F., Comino C., Lanteri S., Portis E., Romani A. "Polyphenolic Composition of Wild and Cultivated Cardoon Leaves", 2007, forthcoming publication).

In the more specifically phytotherapeutic field, it is known that artichoke extracts are obtained by extraction with water or with hydroalcoholic solutions from either fresh or dried leaves. For example, US patent application 2004/0234674 (Eich et al.) describes artichoke leaf extracts characterised by a total content of caffeoylquinic acids (mono- and dicaffeoylquinics of at least 6% with respect to the total quantity of the extract, and a total content of flavonoids of at least 3%, still with respect to the total quantity of extract. According to the description, these products were obtained by means of liquid-liquid extraction of a primary extract from fresh or dried leaves of *Cynara,* in which the extraction solvent is an organic non-aqueous solvent, and the resulting aqueous phase is the one that is recovered.

Still in the same field, the international patent application No. WO 2007/006391 (Indena S.p.A.) describes *Cynara scolymus* extracts obtainable by fractionation on resins, in which the above-surface parts of the plants (including the heads), both in fresh and dried state, are subjected to extraction with a hydroalcoholic solution to which a quantity of cysteine is added in order to obtain a large quantity of cynaropicrin in the final extract, and to maintain this cynaropicrin stable in therapeutic formulations. Sulfurated amino acids are deemed to give rise to adducts that stabilise the sesquiterpene. The hydroalcoholic extracts are then concentrated, and the precipitated substances are separated by filtration, and the resulting solution is concentrated and purified on an adsorption resin. Finally, the desired extract is eluted with ethanol from the resin.

The US patent No. 5780060 (Levy et al.) discloses the use of refined extracts based on polyphenol active ingredients from plants for the production of pharmaceutical, dietetic or food compositions. The concerned plant polyphenols are extracted from the respective plant material and then undergo interfacial crosslinking with a crosslinking agent such as a diacid halide, to give a product in the form of microcapsules.

The US patent No. 6818234 (Nair et al.) discloses the production of human dietary supplements containing naturally occurring phytochemicals which may be useful for pain relief and as anti-inflammatory agents, starting from vegetable products. The concerned phytochemicals are anthocyanins, a specific group of flavonoids which are mainly found in red and blue fruits and flowers. Dietary supplements based on anthocyanins may be obtained, according to this document, by subjecting the starting plant material to extraction to obtain an extract juice, which is then treated by tangential membrane filtration technology (i.e. microfiltration, ultrafiltration and reverse osmosis) in order to obtain purified anthocyanin containing fractions.

The scientific articles by Rafael Llorach et al. (Llorach L. et al., J. Agric. Food Chem., 2002, 50, 3458-3464; Llorach L. et al., Eur. Food Res. Technol., 2005, 220:31-36) disclose processes for the production of extracts from artichoke by-products. In the first case the raw vegetable material was treated both by methanol and by water extraction wherein the respective extractant was kept boiling for 1 h; in the second case the vegetable material was extracted by reflux with boiling water 1:2 w/v for 1 hour, and then cooled and filtered.

Turning back to the agro-food sector, it must be noted that not only consumption of the fresh vegetable, but also - and especially - its processing and packaging produces a high quantity of waste (leaves, stems and waste water). In the artichoke processing industry, in some cases this waste exceeds 60% of the overall vegetable mass. Over and beyond its uses in phytotherapy and cosmetics, the extracts from artichoke and cardoon wastes can be used as additives in order to improve the quality of both animal feeds and human foodstuffs (lowering lipid peroxidation and increasing the health properties of the foods themselves, as well as providing fibre). Moreover, thanks to the antimicrobial properties of the artichoke, the leaf extracts are considered as potential additives in foods for which natural protection is sought (biocontrol) against polluting microbes.

In view of the above, an object of the present invention is to provide a production system enabling the advantageous reuse of the waste products of artichoke production and possibly other vegetable materials or wastes from specifically cultivated plants belonging to the *Cynara* genus, in order to obtain purified extracts, both in powdered and liquid form, useful for their active ingredients to be used in the agro-food industry as well as in nutraceutic, phytotherapeutic, cosmetic and dermatological products.

To this end, a production method has been devised, according to the present invention, for producing extracts of artichoke waste, particularly but not exclusively from the plant leaves, and based on a refining process using membrane-based separation technologies. As is known, these technologies are a safe, reliable and innovative alternative (Best Available Technology) to the traditional solvent-based extraction techniques. These techniques do not introduce contaminating substances (and, in particular, do not use organic solvents), work at room temperature and thus do not thermally damage the matrix, are perfectly sterilizable, and are thus microbiologically safe, and have also been widely tried and tested in the most delicate pharmaceutical preparations.

The proposed process is based on the specialist use of membrane-based separation technologies, applied directly to a raw decoction, in water, of vegetable materials to be treated, after separating the solid material from it by means of mechanic filtration. The filtered liquid undergoes a tangential microfiltration (MF) phase, a tangential ultrafiltration (UF) phase on the previous permeate, whose permeated product yields practically the total amount of nutraceutic active ingredients of *Cynara,* and a reverse osmosis (RO) phase on the UF permeate, which yields a retentate rich in concentrated active ingredients and a permeate, consisting of ultrapure water, that is recycled to the previous extraction phases, and in the preparation of the decoction bath.

For some of the uses envisaged, the RO concentrate is dried or freeze-dried to obtain a stable powder rich in polyphenol antioxidant molecules, in particular, flavonoids (such as the glucosides of luteolin and apigenin) and caffeoylquinic esters (such as chlorogenic acid and cynarin).

Therefore, the present invention specifically provides a process for the production of refined extracts from plants of the *Cynara* genus, comprising, in sequence, the following operations:
a) obtaining a decoction of vegetable material from artichoke waste or from other plants of the *Cynara* genus, in water, by means of hot infusion (that is, at temperatures higher than room temperature, but lower than boiling point) by heating the said material in water, with a weight/volume ratio between 10 and 35% at a temperature between 80 °C and 95 °C, for an overall time ranging between 15 and 45 minutes;
b) mechanically separating the vegetable material obtained in the previous phase from the filtered liquid phase;
c) treating the said filtered liquid phase resulting from operation b) by means of tangential microfiltration (MF), to yield a retentate phase and a permeate phase;
d) treating the permeate coming from the previous operation by means of tangential ultrafiltration (UF), to yield a retentate phase and a permeate phase;
e) treating the permeate coming from operation d) by means of reverse osmosis (RO), to yield a retentate phase rich in purified polyphenol active ingredients and a permeate phase consisting of demineralised water;
the said vegetable material decoction separated from phase b), being depleted of bitter polyphenol compounds, and being reusable as animal feed, and the said MF and UF retentate phases coming from operations c) and d) being reusable in the human food sector.

Preferably, the reverse osmosis concentrate, rich in purified polyphenol active ingredients, is spray-dried or freeze-dried to obtain a stable powder rich in antioxidant molecules.

With specific reference to the case in which the vegetable material to be treated is composed of waste of the artichoke production process, and specifically the leaves, outer bracts and/or stems or even heads that are considered inedible, this material can be used after its separation into the various types of tissues or mixed and, as already noted with reference to the scientific literature published or in publication, will give rise to refined products of an overall different chemical composition. In essence, refined products obtained from stems or from heads have very similar active ingredient compositions, with a prevalence of caffeoylquinic compounds, while leaves and bracts are also rich in flavonoid derivatives.

As already noted, the process of the invention can be applied not only to vegetable material selected from artichoke leaves, outer bracts, stems and heads taken separately or intermixed, but also to the case where the vegetable material comes from cultivated cardoon, *C*. *cardunculus* L. var. *altilis,* or even from wild cardoon, *C*. *cardunculus* L. var. *sylvestris.* In all these cases, the active ingredients recoverable from the ultrafiltration operation belong to correlated polyphenol families, even if, depending on the botanical variety and also on the cultivar and cultivation and harvesting conditions, the tenors of the individual compounds belonging to the main families will be different.

The decoction of the vegetable material is carried out by heating the said material in water (tap or distilled water) at a temperature between 80 °C and 95°C, and preferably 85°C, with a weight/volume ratio between 10 and 35%, for an overall time ranging between 15 and 45 minutes, and preferably about 30 minutes. At the end of heating in an aqueous medium, the decoction is collected by mechanic filtration on a 2-10 mm mesh filter, and preferably a 2 mm stainless steel mesh filter.

The artichoke leaves and stems may undergo a second hot extraction phase to totally remove the residual active ingredients. In this case, after phase b) of mechanical separation, the separated vegetable material undergoes a second decoction phase, followed by a further mechanical separation of the vegetable material obtained, to yield a filtrated liquid phase.

The residual vegetable part that is collected after decoction is depleted or completely devoid (second extraction) of polyphenol and caffeoylquinic compounds, which are particularly bitter, normally present in *Cynara,* and may thus be an interesting raw material for cattle feeds, also by adding other components (forage, purple medic, clover, etc.).

The filtered liquid of the decoction is then cooled to 30 °C and treated with three different membrane-based tangential filtration technologies: microfiltration, ultrafiltration and reverse osmosis.

Operation c) of tangential microfiltration can be carried out with spiral-wound polymer membranes or ceramic membranes, with a molecular size in the range 0.10-3.0 µm. The said spiral-wound polymer membranes are preferably made of polyethersulfone, regenerated cellulose acetate or nylon, while the ceramic ones are preferably made of a tubular ceramic monolith of alumina with an internal coating of zirconia or titanium oxide, of the isoflux type, of a "sunflower" or "dahlia" shape, with 23 or 39 filtration channels.

According to the preferred embodiments of the invention, downstream of the said operation c) of MF a diafiltration is carried out, feeding the membrane with demineralised water (RO permeate), that is added to the MF retentate.

Operation d) of tangential ultrafiltration is preferably carried out with polymer membranes of molecular cut-off between 500 Dalton and 50 kDalton. The polymer membranes for UF are of the spiral type and are made of one of the following materials: polysulfone, polyethersulfone, nylon or regenerated cellulose acetate.

Still according to a preferred solution of the present invention, downstream of operation d) of UF a diafiltration is carried out, feeding the membrane with purified water (RO permeate), obtained from the said operation e) of reverse osmosis, that is added to the UF retentate.

As already noted, the reverse osmosis operation is carried out in order to eliminate the water (permeate) and to concentrate the active ingredients of *Cynara,* and may be conducted with spiral-wound polymer membranes with low or high saline rejection normally at pressures ranging between 0.7 and 5 MPa (7 and 50 bar), and at feed flow rates between 0.5 and 2 m³/h, when 4 x 40 inch (10.16 cm x 101.6 cm) spiral type modules are used.

According to some forms of specific realisations of the invention, the membranes used are polymer membranes of various chemical nature, geometrical shape (flat, hollow-fibre, spiral-wound, tubular, boxed, etc.) and size of the modules.

If desired, the polyphenol active ingredients of the RO concentrate can be directly exploited with the product in liquid form, or they can be dried in order to obtain a powder. Preferably, the said reverse osmosis retentate drying operation is carried out by spray-drying, possibly after adding dextrans or maltodextrins (preferably 30 g/L) to the RO retentate in order to improve the texture and stability of the powder.

The refined extracts based on the purified polyphenol active ingredients directly obtained from the process proposed according to the present invention are advantageously used both for food production and as nutraceutic, pharmaceutical, phytotherapeutic and cosmetic products. In particular, these extracts can be used in even innovative type food products such as special dough for baking products and/or powdered products or, in liquid form, as stabilising additives for foodstuffs, such as for stabilising (in lieu of ascorbic acid) "fresh" vegetal products such as artichoke hearts that are used, unpreserved, in the catering industry.

In solid powdered form, the refined extracts of *Cynara* prepared according to the present invention are useful not only as nutraceutic and pharmaceutical active ingredients, such as for over-the-counter (OTC) products, but also as semi-finished components of antioxidant and anti-free radical activity for cosmetics and dermatological products.

In short, the production system according to the invention is characterised by the following advantageous aspects:
◆ it uses an innovative process enabling the recovery and reuse of all the chemical components resulting from the treatment of the wastes of artichoke production;
◆ it respects the environment, also by the integrated reuse of secondary effluents, including osmotised water;
◆ it avoids the use of organic solvents in the extraction, which would negatively affect the use of the recovered active ingredients in foodstuffs and pharmaceuticals;
◆ it enables the development of an industrial process of a scale consistent with the quantities of raw material available in the national territory owing to the membrane technologies, which are modular and thus easily adaptable to any scale of production;
◆ it allows using a range of purified, concentrated active ingredients, formulated in stabilised form.

The specific characteristics of the present invention, as well as its advantages and relative operational modalities, will be more evident with reference to the detailed description presented merely for exemplificative purposes below, along with the results of the experimentations carried out on it. The diagrams of the proposed process and some experimental results are also illustrated in the attached drawings, wherein:
Figure 1 shows a block diagram of the extraction and refining process from vegetal material of the *Cynara* genus proposed according to the present invention;
Figure 2 shows the trend over time of the permeate flow through the microfiltration (MF) membrane of the Example.;
Figure 3 shows the trend over time of the permeate flow through the ultrafiltration (UF) membrane of the Example;
Figure 4 shows the HPLC chromatogram of the artichoke extract powder obtained as the final product from the process of the Example, with the legend of the polyphenol compounds present; and
Figure 5 shows the HPLC chromatogram of the powder obtained from a wild cardoon extract, with the legend of the polyphenol compounds present.

### EXAMPLE

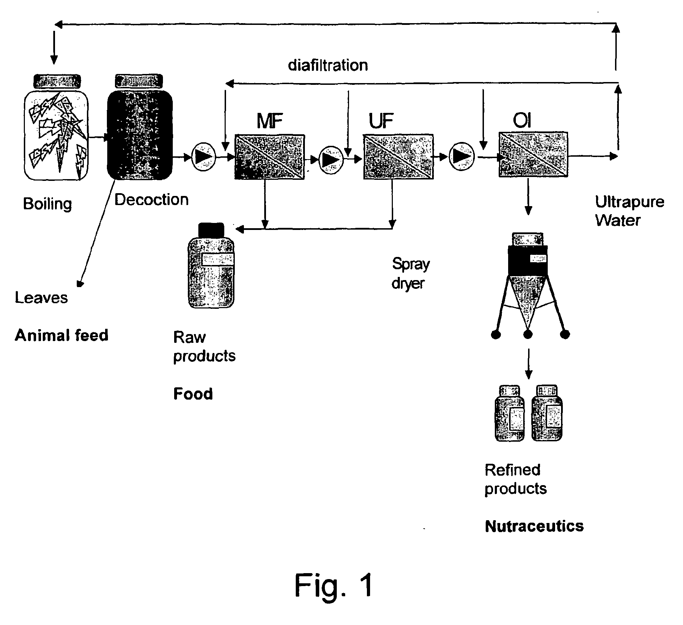

The overall scheme of the process applied is the same as the one shown in **Figure 1**.

### Decoction of the vegetal material

The aqueous extract of artichoke leaves is obtained by heating the vegetal material (leaves, bracts, stems, the heads considered inedible for their size, etc.) in water at 85 °C for about 30 minutes, avoiding boiling.

In this case, 25 kg of fresh vegetal material (leaves) was used in 100 L of tap water (25% dry weight), with no particular requirements of chemical purity. The decoction. solution is separated from the leaves by simple filtering on 2 mm wire mesh and then fed to tangential microfiltration. This solution was characterised as regards the biophenol content of the starting extract (the input datum is necessary both for standardising the extraction conditions and for defining the characteristics of the finished product).

### Microfiltration of the decoction, with 0.14 µm molecular cut-off

The aqueous extract was microfiltered (MF) with alumina tubular ceramic membranes, internally coated with zirconia, of the isoflux type by Tami (France), and of a "sunflower" shape (23 channels, 3.6 mm in diameter) with a porosity of 0.14 µm and filtering surface of 0.35 m².

The process is carried out with a pilot apparatus of the ENEA Casaccia laboratories that uses two ceramic membranes in parallel of the aforesaid type. The process parameters of the MF trial are reported in Table 1 below.

**Table 1. Process parameters of the MF 0.14 µm trial**

| **Process parameters** | **Value** |
|---|---|
| Feed flow rate | 9.5 m³/hour |
| Transmembrane pressure | 0.135 M Pa (1.35 bar) |
| Temperature | 20-29 °C |
| Flow speed | 5.6 m/s |
| VCR (volumetric concentration ratio) | ca. 3.5 |

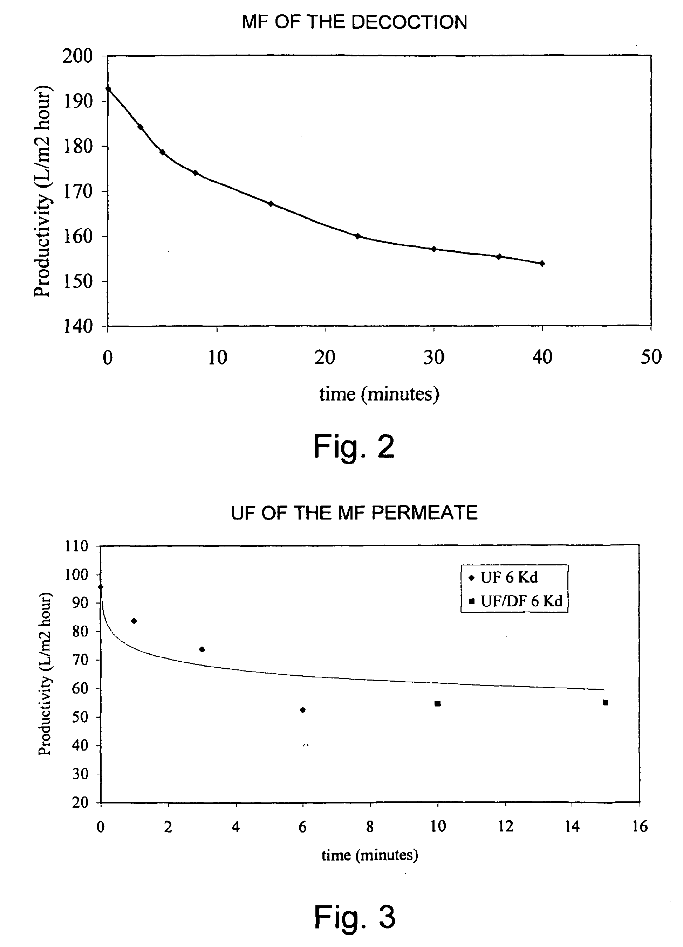

The diagram of the attached **Figure 2** shows the production curve in terms of litres produced over time and with respect to the filtering surface of the MF 0.14 µm trial.

Once the VCR (volumetric concentration ratio) of about 3.5 is reached, the 28 litres of MF 0.14 µm concentrate were added with 23 litres of osmotised water (MF/DF), continuing the filtering in order to increase the extraction of the molecules of interest in the permeate. The MF/DF process with a 0.14 micron membrane yielded 26.5 litres of permeate. The final volume of concentrate of 0.14 micron MF/DF is of about 25 litres. The mean flow rate of the permeate in DF increases to values of about 170 (L/m²h).

### Ultrafiltration of the MF permeate, with 6 kD molecular cut-off

This operation uses, as feeder, a solution of 97 L, + hold-up volume (ca. 7 litres), for a total of about 104 L.

The UF operation aims to eliminate compounds of high molecular weight (proteins, colloidal compounds, pectins, waxes, fragments of cell walls, etc.).

A spiral-wound polyethersulfone polymer membrane (Osmonics, USA) is used, with a molecular size of 6 kD, 28 mil spacer and filtering surface of 8.36 m².

The process parameters of the UF trial are reported in Table 2 below.

**Table 2. Process parameters of the UF 6 kD trial**

| **Process parameters** | **Value** |
|---|---|
| Feed flow rate | 4.4 m³/hour |
| Transmembrane pressure | 0.47 MPa (4.7 bar) |
| Temperature | 22-24 °C |
| Flow rate | 0.25 m/s |
| VCR (volumetric concentration ratio) | ca. 4.1 |

Once the VCR of about 4.1 is reached, to the 24 litres of concentrate (+ hold-up volume) is added 21 litres of osmotised water four times (making a total of 84 litres added), continuing the filtering (UF/DF) to increase the yield. A total of 95 litres of permeate of UF/DF 6 kD are obtained and 13 litres + hold-up volume of the UF/DF 6 kD concentrate.

The diagram of **Figure 3** attached shows the production curve in terms of litres produced over time and with respect to the filtering surface of the UF and UF/DF 6 kD trials.

### Reverse osmosis (RO) operation on the UF permeate

The UF permeates and UF/DF ones are treated in RO in order to concentrate the product rich in antioxidant substances, and to eliminate the water, as permeate.

80 litres of UF 6 kD permeate along with 95 litres of UF/DF 6 kD permeate (+ hold-up volume of about 10 litres), for a total of 185 L, were concentrated in RO with a pilot apparatus equipped with a polymer module made of composite spiral-wound high saline rejection polyamide, of the DE SAL company (USA), with a filtering surface of 7.0 m². The process parameters are reported in Table 3 below.

**Table 3. Process parameters of the RO trial**

| **Process parameters** | **Value** |
|---|---|
| Feed flow rate | 0.65-0.60 m³/hour |
| Operating pressure | 2.0-2.6 MPa (20-26 bar) |
| Temperature | 22-27 °C |
| Mean permeate flow | 22 (L/m²h) |
| Salinity expressed in mg/l | 0-56 |
| VCR (volumetric concentration ratio) | 15.0 |

Membrane productivity remains constant in the region of 18.8 L/m² per hour throughout the trial.

During the trial, the pressure - initially set at 2.0 MPa (20 bar) - automatically rises in the apparatus up to 2.6 MPa (26 bar) after 40 minutes, owing to an increase in permeate salinity. For this reason, productivity at 2.6 MPa (26 bar) increases to 23.14 L/m² per hour.

The RO trial lasted for a total of 65 minutes, in which no loss of permeability of the module was observed, which means that this membrane can operate without washing for long periods (months).

The liquid concentrated product is bright green due to the presence of chlorophyll.

As shown by the process diagram of **Figure 1**, the RO permeate is reused in order to prepare the new extraction bath, that is, to prepare the decoction, avoiding the use of tap or well water.

### Drying of the RO concentrate

The liquid product obtained as a retentate of the preceding phase is then turned into powder by a spray-drying process by means of a type ICF Lab 25 laboratory spray-dryer with an evaporation capacity of 500 ml/h.

The working conditions of the spray-dryer are the following:
pump flow rate: 5 ml/minute;
spray-drying chamber temperature: 90°C;
exiting air temperature: 85°C;
vacuum: 3 kPa (30 mbar).

The RO concentrate undergoing spray-drying yielded a yellowish green solid powdery product containing about 5% humidity. This powder may be further dried in an oven, and must be protected from humidity, given its hygroscopic nature.

The possibility of obtaining solid extracts of various concentrations was also studied, with the addition of dextrans or maltodextrins, or inert powders such as silica, according to the purpose.

### Membrane washing cycle

At the end of the work cycle, the membrane modules must be reconditioned in order to carry out the next work cycle. This allows removing any deposits forming on the membrane in order to restore the best permeate flows, that is, the productivity of these filters. To wash MF ceramic membranes it is necessary to first rinse the module with tap water for 5 minutes and then chemical wash it with a base solution (0.5 M soda) at 35°C for 25 minutes in continuous mode. A second wash is then carried out with water to eliminate the chemical reactive until neutralisation of the washing solution is obtained.

For UF, the rinsing operation is to be carried out with tap water for 5-10 minutes, without recycling the water, followed by treatment with 0.25 M soda for 15 minutes. At the end of this operation, the module is washed with tap water and its permeability is checked.

For RO, the washing protocol simply envisages rinsing with tap water, followed by washing with distilled water.

### Chromatographic analysis of the dried RO concentrate

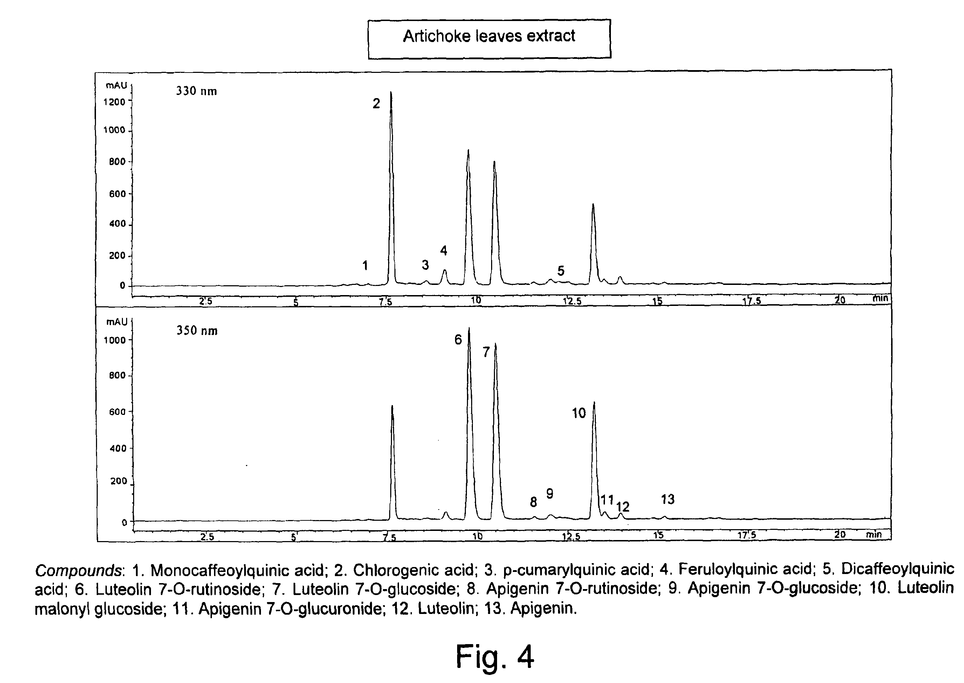

The chromatogram of the powder obtained by drying the RO concentrate is shown in **Figure 4** attached.

Each molecule is quantified individually and the titre of the extract is defined as total caffeoylquinic derivatives and as total flavonoids, besides the total polyphenols present.

Table 4 below shows the quantitative results of the previously identified individual molecules relative to the starting aqueous extract and to the RO concentrate after the membrane-based separation process. The data are obtained by HPLC/DAD analysis.

**TABLE 4**

| | | |
|---|---|---|
| Individual compounds in artichoke extracts, ppm | | |

| | **Aqueous extract** | **RO Conc.** |
|---|---|---|
| Chlorogenic acid | 262.7 | 466.7 |
| MCQs (mono-caffeoylquinics) | 496.0 | 895.4 |
| Cynarin | 46.0 | 81.3 |
| Other DCQs (di-caffeoylquinics) | 42.0 | 38.0 |
| luteolin 7 -O-rutinoside | 47.9 | 63.9 |
| luteolin 7-O-glucoside | 14.0 | 21.0 |
| luteolin 7-O-malonyl glucoside | 8.5 | 13.0 |
| Luteolin | 2.9 | 3.3 |
| **TOTAL POLYPHENOLS** | **920.7** | **1582.6** |

The anti-free radical capacity of the extract that can be correlated to the antioxidant properties is evaluated spectrophotometrically by means of the stable radical DPPH. Previous studies on artichoke leaves or bracts showed a dose-dependent effect. also correlated to the type of molecules present, on human LDL (Coinu et al. 2006, *loc*. *cit.*)*.*

Most of the cynarin present in the aqueous extracts of artichoke derives from 1,3-dicaffeoylquinic acid, initially found in the fresh plant following intramolecular trans-esterification caused by heating (Panizzi L. & Scarpati M.L., Gazz. Chim. Ital., 95, 71-82, 1965). 1,3-dicaffeoylquinic acid is the largest component of dicaffeoylquinic esters in hydroalcoholic extracts obtained cold, where the largest compound is the mono-substituted 5-caffeoylquinic derivative (chlorogenic acid).

Table 5 below reports the quantitative results of the single compounds analysed by HPLC/DAD obtained by spray-drying from the RO concentrate, considering Sample 1 as the powder as it is, and Sample 2 after adding 25 g/L of dextran to the RO concentrate.

**TABLE 5**

| Individual powder compounds of dried RO concentrate, mg/g | | |
|---|---|---|
| | **Sample 1** | **Sample 2** |
| Chlorogenic acid | 34.4 | 17.2 |
| MCQs | 64.8 | 32.6 |
| Cynarin | 5.5 | 2.9 |
| Other DCQs | 3.1 | 1.0 |
| Luteolin 7-O-rutinoside | 5.0 | 2.5 |
| Luteolin 7-O-glucoside | 2.0 | 1.0 |
| Luteolin 7-O-malonyl glucoside | 1.2 | 0.5 |
| Luteolin | 0.3 | 0.2 |
| **TOTAL POLYPHENOLS** | **116.3** | **57.9** |

Both the starting aqueous extract and all the intermediate solutions, including the RO concentrate and spray-drying product, were HPLC/DAD/MS analysed in order to establish the content in the caffeoylquinic derivatives and flavonoids present. The analytical and quantification conditions were standardised.

The previous results show how this type of process can yield both liquid and powder extracts of different contents in polyphenol compounds. The extracts can, in fact, come from a mixture of all the waste material of artichoke processing or it is possible to hypothesise the devising of a compressed air system that separates the stems and heads from the lighter tissues, consisting of bracts and leaves. In this way, processing the heads and stems will yield an extract rich in caffeoylquinic derivatives, while an extract rich in these compounds and in flavonoids can be obtained from the bracts and leaves.

The same technologies and extraction conditions can be applied to both cultivated and wild cardoon tissues in order to obtain extracts that are quantitatively and qualitatively different from those of the artichoke.

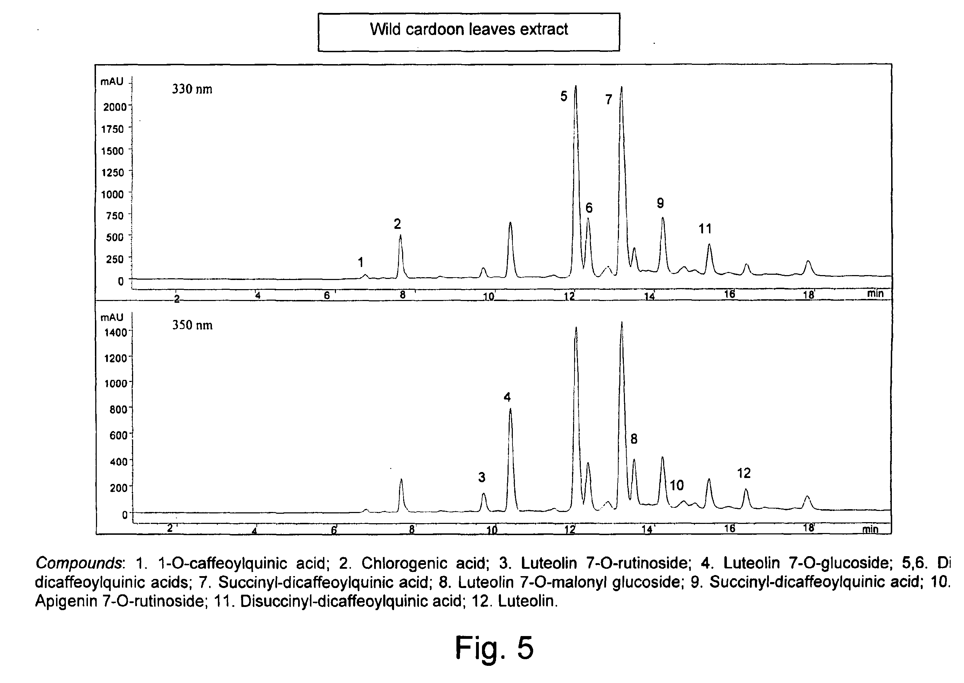

Specifically, the attached **Figure 5** shows a HPLC chromatogram of a hydroalcoholic extract of wild cardoon, which yields the specific proportions of the various polyphenol compounds present.

It is also worth specifying that the titre in the bioactive components of the previous extracts can be expressed both as the total content obtained spectrophotometrically and as the content in the three different subclasses (mono- and di-caffeoylquinic esters and flavonoids) evaluated by means of HPLC/DAD. As described above, each fraction may also be evaluated for the specific anti-free radical activity by means of DPPH that can be correlated to the antioxidant properties of the extract itself.

As may be noted from the above, one of the main advantages of the present invention is the possibility of valorising and reusing the large quantities of vegetal biomass resulting as waste from artichoke production, thereby obtaining products of high added value for the food and pharmaceutical industry, by means of a process that respects the environment and ecosystem. negative anaerobic/aerobic bacteria.

The present invention has been disclosed with particular reference to some specific embodiments thereof, but it should be understood that modifications and changes may be made by the persons skilled in the art without departing from the scope of the invention as defined in the appended claims.

## Claims

1. A process for the production of refined extracts from plants of the *Cynara* genus, comprising, in sequence, the following operations:
a) obtaining a decoction of vegetable material from artichoke waste or from other plants of the *Cynara* genus, by heating the said material in water, with a weight/volume ratio between 10 and 35% at a temperature between 80°C and 95°C, for an overall time ranging between 15 and 45 minutes;
b) mechanically separating the vegetable material obtained in the previous phase from a filtered liquid phase;
c) treating the said filtered liquid phase resulting from the previous by means of tangential microfiltration (MF), to yield a retentate phase and a permeate phase;
d) treating the permeate coming from the previous operation by means of tangential ultrafiltration (UF), to yield a retentate phase and a permeate phase;
e) treating the permeate coming from operation d) by means of reverse osmosis (RO), to yield a retentate phase rich in purified polyphenol active ingredients and a permeate phase consisting of demineralised water;
the said vegetable material decoction obtained from the mechanical separation, being depleted of bitter polyphenol compounds, and being reusable as animal feed, and the said MF and UF retentate phases coming from operations c) and d) being reusable in the human food sector.

2. A process according to claim 1, wherein the said reverse osmosis retentate phase rich in purified polyphenol active ingredients obtained from operation e) undergoes spray-drying or freeze-drying.

3. A process according to claims 1 or 2, wherein the said vegetable material from which the decoction is obtained derives from the leaves, outer bracts, stems and heads of the artichoke, *Cynara scolymus,* either taken separately or intermixed.

4. A process according to claims 1 or 2, wherein the said vegetable material from which the decoction is obtained derives from the leaves, outer bracts, stems and heads of the cultivated cardoon, *C*. *cardunculus* L. var. *altilis,* either taken separately or intermixed.

5. A process according to claims 1 or 2, wherein the said vegetable material from which the decoction is obtained derives from the leaves, outer bracts, stems and heads of the wild cardoon, *C*. *cardunculus* L. var. *sylvestris,* either taken separately or intermixed.

6. A process according to claim 5, wherein the said vegetal material is heated at a temperature of 85 E for an overall time period of 30 minutes.

7. A process according to any one of claims 1-6, wherein the said mechanical separation operation b) of the vegetable material obtained from the previous phase is carried out by mechanical filtration on a 2-10 mm wire mesh filter.

8. A process according to any one of claims 1-7, wherein after the said phase b) of mechanical separation, the separated vegetable material undergoes a second decoction phase followed by a further mechanical separation of the vegetable material obtained, in order to yield a filtered liquid phase.

9. A process according to any one of claims 1-8, wherein the said operation c) of tangential microfiltration is carried out by means of spiral-wound polymer membranes or ceramic membranes with a molecular cut-off in the range 0.10-3.0 µm.

10. A process according to claim 9, wherein the said polymer membranes are made of polyethersulfone, regenerated cellulose acetate or nylon.

11. A process according to claim 9, wherein the said ceramic membranes for microfiltration are made of a tubular ceramic monolith in alumina with an internal coating of zirconia or titanium oxide.

12. A process according to any one of claims 1-11, wherein downstream of the said operation c) of tangential microfiltration (MF) a diafiltration is carried out, feeding the membrane with demineralised water obtained from the said operation e) of reverse osmosis, that is added to the MF retentate.

13. A process according to any one of claims 1-12, wherein the said operation d) of tangential ultrafiltration is carried out with polymer membranes with a molecular cut-off between 500 Dalton and 50 kDalton.

14. A process according to claim 13, wherein the said polymer membranes for UF are spiral-wound.

15. A process according to any one of claims 1-14, wherein downstream of the said operation d) of tangential ultrafiltration (UF) a diafiltration is carried out, feeding the membrane with demineralised water obtained from the said operation e) of reverse osmosis, that is added to the UF retentate.

16. A process according to any one of claims 1-15, wherein the said operation e) of reverse osmosis is carried out with spiral-wound polymer membranes with low or high saline rejection at an operating pressure between 0.7 and 5 MPa (7 and 50 bar).

17. A process according to claim 16, wherein the said spiral-wound polymer membranes have a mesh spacer with thickness ranging between 20 and 38 mil (0.51-0.97 mm), and size of the filtering modules 4 inches in diameter x 40 inches in length (10.16 cm x 101.6 cm), 6 inches in diameter x 40 inches in length (15.24 cm x 101.6 cm), or 8 inches in diameter x 40 inches in length (20.32 cm x 101.6 cm).

18. A process according to claims 16 or 17, wherein the said polymer membranes are flat, spiral-wound, hollow-fibre or boxed membranes.

19. A process according to any one of claims 2-17, wherein the said drying operation of the reverse osmosis (RO) retentate is carried out by spray-drying.

20. A process according to claim 19, wherein the said spray-drying operation is carried out after adding dextrans or maltodextrins to the said RO retentate.

21. A raw material for cattle feeds consisting of said vegetable material decoction obtained from the mechanical separation of step b) of the process of claim 1.

22. A raw material for human food preparations consisting of said MF and UF retentate phases obtained from operations c) and d) of the process of claim 1.

## Patentansprüche

1. Verfahren zur Herstellung raffinierter Extrakte aus Pflanzen der Gattung *Cynara,* das der Reihe nach folgende Arbeitsgänge umfasst:
a) Gewinnen eines Pflanzenmaterialdekoktes aus Artischockenresten oder anderen Pflanzen der Gattung *Cynara* durch Erhitzen des Materials in Wasser in einem Gewicht/Volumen-Verhältnis zwischen 10 und 35% und bei einer Temperatur zwischen 80°C und 95°C für eine Gesamtdauer von 15 bis 45 Minuten,
b) mechanisches Trennen des in der vorangegangenen Phase gewonnenen Pflanzenmaterials von einer gefilterten flüssigen Phase,
c) Behandeln der sich so ergebenden gefilterten flüssigen Phase mit Hilfe tangentialer Mikrofiltration (MF), um eine Retentatphase und eine Permeatphase zu erhalten,
d) Behandeln des Permeats aus dem vorangegangenen Arbeitsgang mit Hilfe tangentialer Ultrafiltration (UF), um eine Retentatphase und eine Permeatphase zu erhalten,
e) Behandeln des Permeats aus Arbeitsgang d) mit Hilfe von Reversosmose (RO), um eine Retentatphase zu erhalten, die reich an gereinigten Polyphenolwirkstoffen ist, und eine Permeatphase zu erhalten, die aus entmineralisiertem Wasser besteht,
wobei das durch die mechanische Trennung gewonnene Pflanzenmaterialdekokt arm an bitteren Polyphenolverbindungen ist und als Tierfutter wiederverwendbar ist und die MF- und UF-Retentatphasen aus den Arbeitsgängen c) und d) in der Lebensmittelbranche wiederverwendbar sind.

2. Verfahren nach Anspruch 1, bei dem die im Arbeitsgang e) gewonnene, an gereinigten Polyphenolwirkstoffen reiche Retentatphase aus der Reversosmose sprüh- oder gefriergetrocknet wird.

3. Verfahren nach Anspruch 1 oder 2, bei dem das Pflanzen material, aus dem das Dekokt gewonnen wird, von den Blättern, Hüllblättern, Stielen und Köpfen der Artischocke, *Cynara scolymus,* stammt, die entweder getrennt oder miteinander vermischt genommen sind.

4. Verfahren nach Anspruch 1 oder 2, bei dem das Pflanzenmaterial, aus dem das Dekokt gewonnen wird, von den Blättern, Hüllblättern, Stielen und Köpfen der kultivierten Kardone, *C*. *cardunculus* L., var. *altilis,* stammt, die entweder getrennt oder miteinander vermischt genommen sind.

5. Verfahren nach Anspruch 1 oder 2, bei dem das Pflanzenmaterial, aus dem das Dekokt gewonnen wird, von den Blättern, Hüllblättern, Stielen und Köpfen der wilden Kardone, *C*. *cardunculus* L., var. *sylvestris,* stammt, die entweder getrennt oder miteinander vermischt genommen sind.

6. Verfahren nach Anspruch 5, bei dem das Pflanzenmaterial für eine Gesamtdauer von 30 Minuten bei einer Temperatur von 85°C erhitzt wird.

7. Verfahren nach einem der Ansprüche 1 bis 6, bei dem der Arbeitsgang b) der mechanischen Trennung des aus der vorangegangenen Phase gewonnenen Pflanzenmaterials durch mechanische Filtration an einem Drahtgewebefilter mit 2 bis 10 mm Maschenweite durchgeführt wird.

8. Verfahren nach einem der Ansprüche 1 bis 7, bei dem das getrennte Pflanzenmaterial nach der Phase b) der mechanischen Trennung einer zweiten Dekoktionsphase unterzogen wird, auf die eine weitere mechanische Trennung des gewonnenen Pflanzenmaterials folgt, um eine gefilterte flüssige Phase zu erhalten.

9. Verfahren nach einem der Ansprüche 1 bis 8, bei dem der Arbeitsgang c) der tangentialen Mikrofiltration mit Hilfe spiralförmig gewickelter Polymermembranen oder Keramikmembranen mit einer molekularen Ausschlussgrenze im Bereich von 0,10 bis 3,0 µm durchgeführt wird.

10. Verfahren nach Anspruch 9, bei dem die Polymermembranen aus Polyethersulfon, regeneriertem Celluloseacetat oder Nylon hergestellt sind.

11. Verfahren nach Anspruch 9, bei dem die Keramikmembranen für die Mikrofiltration aus einem röhrenförmigen Keramikmonolithen in Aluminiumoxid mit einer Innenbeschichtung aus Zirkonium- oder Titanoxid hergestellt sind.

12. Verfahren nach einem der Ansprüche 1 bis 11, bei dem nach dem Arbeitsgang c) der tangentialen Mikrofiltration (MF) eine Diafiltration durchgeführt wird, indem der Membran aus dem Arbeitsgang e) der Reversosmose gewonnenes entmineralisiertes Wasser zugeführt wird, das zum MF-Retentat hinzugefügt wird.

13. Verfahren nach einem der Ansprüche 1 bis 12, bei dem der Arbeitsgang d) der tangentialen Ultrafiltration mit Polymermembranen mit einer molekularen Ausschlussgrenze zwischen 500 Dalton und 50 kDalton durchgeführt wird.

14. Verfahren nach Anspruch 13, bei dem die Polymermembranen für die UF spiralförmig gewickelt sind.

15. Verfahren nach einem der Ansprüche 1 bis 14, bei dem nach dem Arbeitsgang d) der tangentialen Ultrafiltration (UF) eine Diafiltration durchgeführt wird, indem der Membran aus dem Arbeitsgang e) der Reversosmose gewonnenes entmineralisiertes Wasser zugeführt wird, das zum UF-Retentat hinzugefügt wird.

16. Verfahren nach einem der Ansprüche 1 bis 15, bei dem der Arbeitsgang e) der Umkehrosmose mit spiralförmig gewickelten Polymermembranen mit geringer oder starker Salzrückhaltung bei einem Betriebsdruck zwischen 0,7 und 5 MPa (7 und 50 bar) durchgeführt wird.

17. Verfahren nach Anspruch 16, bei dem die spiralförmig gewickelten Polymermembranen einen Gewebeabstandhalter mit einer Dicke zwischen 20 und 38 mil (0,51-0,97 mm) und eine Filtermodulgröße von 4 Zoll Durchmesser x 40 Zoll Länge (10,16 cm x 101,6 cm), 6 Zoll Durchmesser x 40 Zoll Länge (15,24 cm x 101,6 cm) oder 8 Zoll Durchmesser x 40 Zoll Länge (20,32 cm x 101,6 cm) aufweisen.

18. Verfahren nach Anspruch 16 oder 17, bei dem es sich bei den Polymermembranen um flache, spiralförmig gewickelte, Hohlfaser- oder Plattenmembranen handelt.

19. Verfahren nach einem der Ansprüche 2 bis 17, bei dem der Trocknungsarbeitsgang an dem Reversosmose-Retentat (RO-Retentat) durch Sprühtrocknen durchgeführt wird.

20. Verfahren nach Anspruch 19, bei dem der Sprühtrocknungsarbeitsgang nach dem Hinzufügen von Dextranen oder Maltodextrinen zum RO-Retentat durchgeführt wird.

21. Rohmaterial für Viehfutter, das aus dem Pflanzenmaterialdekokt besteht, welches durch die mechanische Trennung in Schritt b) im Verfahren nach Anspruch 1 gewonnen wird.

22. Rohmaterial für Lebensmittelzubereitungen, das aus den MF- und UF-Retentatphasen besteht, welche durch die Arbeitsgänge c) und d) des Verfahrens nach Anspruch 1 gewonnen werden.

## Revendications

1. Procédé de production d'extraits raffinés de plantes du genre *Cynara,* comprenant, à la suite, les opérations suivantes :
a) l'obtention d'une décoction de matériau végétal de déchets d'artichaut ou d'autres plantes du genre *Cynara,* en chauffant ledit matériau dans de l'eau, avec un rapport en poids/volume compris entre 10 et 35 %, à une température comprise entre 80 °C et 95 °C et pendant une durée totale dans la plage de 15 à 45 minutes ;
b) la séparation mécanique du matériau végétal obtenu lors de l'étape précédente à partir d'une phase liquide filtrée ;
c) le traitement de ladite phase liquide filtrée provenant de l'étape précédente au moyen d'une microfiltration (MF) tangentielle, pour donner une phase de rétentat et une phase de perméat ;
d) le traitement du perméat provenant de l'opération précédente au moyen d'une ultrafiltration (UF) tangentielle, pour donner une phase de rétentat et une phase de perméat ;
e) le traitement du perméat provenant de l'opération d) au moyen d'une osmose inverse (RO), pour donner une phase de rétentat riche en ingrédients actifs de polyphénol purifié et une phase de perméat composée d'eau déminéralisée ;
ladite décoction de matériau végétal obtenue à partir de la séparation mécanique étant appauvrie en composés polyphénol amers, et étant réutilisable en tant que nourriture pour animaux, et lesdites phases de rétentat MF et UF provenant des opérations c) et d) étant réutilisables dans le secteur de l'alimentation humaine.

2. Procédé selon la revendication 1, dans lequel ladite phase de rétentat riche en ingrédients actifs de polyphénol purifié obtenue par osmose inverse à partir de l'opération e) subit un séchage par pulvérisation ou une lyophilisation.

3. Procédé selon la revendication 1 ou 2, dans lequel ledit matériau végétal à partir duquel ladite décoction est obtenue est issu des feuilles, des bractées externes, des pédoncules et des têtes de l'artichaut, *Cynara scolymus,* pris séparément ou mélangés.

4. Procédé selon la revendication 1 ou 2, dans lequel ledit matériau végétal à partir duquel la décoction est obtenue est issu des feuilles, des bractées externes, des pédoncules et des têtes du cardon cultivé, *C*. *cardunculus* L. var. *altilis*, pris séparément ou mélangés.

5. Procédé selon la revendication 1 ou 2, dans lequel ledit matériau végétal à partir duquel la décoction est obtenue est issu des feuilles, des bractées externes, des pédoncules et des têtes du cardon sauvage, *C*. *cardunculus* L. var. *sylvestris,* pris séparément ou mélangés.

6. Procédé selon la revendication 5, dans lequel ledit matériau végétal est chauffé à une température de 85 °C pendant une durée totale de 30 minutes.

7. Procédé selon l'une quelconque des revendications 1 à 6, dans lequel ladite opération b) de séparation mécanique du matériau végétal obtenu à l'étape précédente est réalisée par filtration mécanique sur un filtre de maille 2 - 10 mm.

8. Procédé selon l'une quelconque des revendications 1 à 7, dans lequel après ladite phase b) de séparation mécanique, le matériau végétal séparé subit une seconde phase de décoction suivie par une autre séparation mécanique du matériau végétal obtenu, afin de donner une phase liquide filtrée.

9. Procédé selon l'une quelconque des revendications 1 à 8, dans lequel ladite opération c) de microfiltration tangentielle est effectuée au moyen de membranes polymères en serpentin ou de membranes céramiques présentant un seuil de rétention des molécules dans la plage de 0,10 à 3,0 µm.

10. Procédé selon la revendication 9, dans lequel lesdites membranes polymères sont faites de polyéthersulfone, d'acétate de cellulose régénérée ou de nylon.

11. Procédé selon la revendication 9, dans lequel lesdites membranes céramiques de microfiltration sont faites d'un monolithe céramique tubulaire en alumine pourvu d'un revêtement interne de zirconium ou d'oxyde de titane.

12. Procédé selon l'une quelconque des revendications 1 à 11, dans lequel, en aval de ladite opération c) de microfiltration (MF) tangentielle, est réalisée une diafiltration en alimentant la membrane avec de l'eau déminéralisée, obtenue à partir de ladite opération e) d'osmose inverse, qui est ajoutée au rétentat de MF.

13. Procédé selon l'une quelconque des revendications 1 à 12, dans lequel ladite opération d) d'ultrafiltration tangentielle est réalisée avec des membranes polymères présentant un seuil de rétention des molécules compris entre 500 daltons et 50 kdaltons.

14. Procédé selon la revendication 13, dans lequel lesdites membranes polymères d'UF sont en serpentin.

15. Procédé selon l'une quelconque des revendications 1 à 14, dans lequel, en aval de ladite opération d) d'ultrafiltration (UF) tangentielle, est réalisée une diafiltration en alimentant la membrane avec de l'eau déminéralisée, obtenue à partir de ladite opération e) d'osmose inverse, qui est ajoutée au rétentat d'UF.

16. Procédé selon l'une quelconque des revendications 1 à 15, dans lequel ladite opération e) d'osmose inverse est réalisée avec des membranes polymères en serpentin présentant un taux de rejet salin faible ou élevé à une pression de service comprise entre 0,7 et 5 MPa (7 et 50 bar).

17. Procédé selon la revendication 16, dans lequel lesdites membranes polymères en serpentin ont une toile à ouverture de maille présentant une épaisseur comprise entre 20 et 38 mil (0,51 et 0,97 mm), et une taille des modules de filtration de 4 pouces de diamètre x 40 pouces de long (10,16 cm x 101,6 cm), 6 pouces de diamètre x 40 pouces de long (15,24 cm x 101,6 cm) ou 8 pouces de diamètre x 40 pouces de long (20,32 cm x 101,6 cm).

18. Procédé selon la revendication 16 ou 17, dans lequel lesdites membranes polymères sont des membranes plates, en serpentin, à fibres creuses ou en caisson.

19. Procédé selon l'une quelconque des revendications 2 à 17, dans lequel ladite opération de séchage du rétentat de l'osmose inverse (RO) est effectuée par séchage par pulvérisation.

20. Procédé selon la revendication 19, dans lequel ladite opération de séchage par pulvérisation est effectuée après ajout de dextranes ou de maltodextrines audit rétentat de RO.

21. Matériau brut pour l'alimentation du bétail composé de ladite décoction de matériau végétal obtenue à partir de la séparation mécanique de l'étape b) du procédé de la revendication 1.

22. Matériau brut pour préparations alimentaires humaines, composé desdites phases de rétentat MF ou UF obtenues à partir des opérations c) et d) du procédé de la revendication 1.
